Europäisches Patentamt

European Patent Office

Office européen des brevets

(11) Publication number: **0 381 776 B1**

# EUROPEAN PATENT SPECIFICATION

(45) Date of publication of patent specification: **06.04.94**  (51) Int. Cl.⁵: **C12M 1/40**, //C12M1/08

(21) Application number: **89908876.9**

(22) Date of filing: **01.08.89**

(86) International application number:
**PCT/JP89/00787**

(87) International publication number:
**WO 90/01539 (22.02.90 90/05)**

(54) **DOUBLE-CYLINDER TYPE BIO-REACTOR.**

(30) Priority: **10.08.88 JP 199392/88**

(43) Date of publication of application:
**16.08.90 Bulletin 90/33**

(45) Publication of the grant of the patent:
**06.04.94 Bulletin 94/14**

(84) Designated Contracting States:
**BE DE FR GB NL**

(56) References cited:
**JP-B- 569 316**
**JP-U- 6 177 800**
**JP-U-61 150 498**
**JP-Y- 627 116**

(73) Proprietor: **KIRIN BEER KABUSHIKI KAISHA**
**26-1, Jingumae 6-chome**
**Shibuya-ku Tokyo 150(JP)**

(72) Inventor: **SUZUKI, Akira, c/o Kirin Beer K.K.**
**26-1, Jingumae 6-chome**
**Shibuya-ku, Tokyo 150(JP)**
Inventor: **NAGARA, Akira Kirin Beer Kabushiki**
**Kaisha**
**26-1, Jingumae**
**6-chome Shibuya-ku Tokyo 150(JP)**
Inventor: **MITSUI, Shunsuke Kirin Beer**
**Kabushiki Kaisha**
**26-1, Jingumae**
**6-chome Shibuya-ku Tokyo 150(JP)**

(74) Representative: **Lippert, Hans, Dipl.-Ing. et al**
**Reichel und Reichel**
**Parkstrasse 13**
**D-60322 Frankfurt (DE)**

**Description**

TECHNICAL FIELD

The present invention relates to a bioreactor of the double-cylinder type, and in particular, to a bioreactor of the double-cylinder type in which the heat-transfer surface is enlarged at the pre-reaction portion of the reaction tank, and in which the gas removal is performed to the maximum extent possible, and which has a simple structure and good operability.

BACKGROUND ART

Conventionally, cells, enzymes that include cells, other types of substances that include cells, and bacteria (hereinafter termed the biological catalyst) have been used as catalysts in bioreactors where a reaction chamber is used to obtain a predetermined biological substance, and in general, the filled-tank reactors have been used, as the plug-flow type reactors.

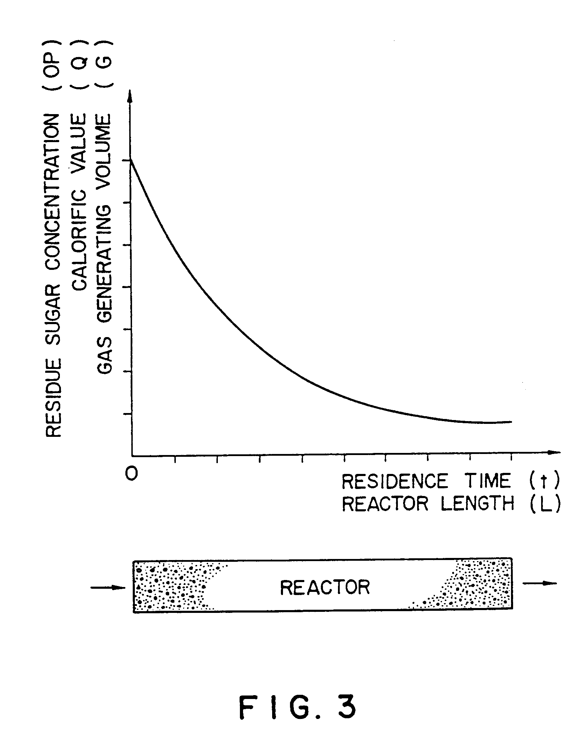

FIG. 3 indicates a mock reaction that occurs in a plug-flow reactor of this type, and there are many situations where the reaction becomes complex because of resistance accompanying secondary reactions and metastasis caused by the reaction system. However, basically, these reactions are according to the Michaelis-Menten equation shown below.

$$r = \frac{V_{max} \cdot [S]}{K_m + [S]} \qquad \cdots (1)$$

Here, r is the reaction speed, $V_{max}$ is the maximum reaction speed, [S] is the substance concentration and $K_m$ is a constant.

However, the following approximation holds for any reaction system.

$$[S] = S_0 (1 - e^{-\frac{t}{A}}) \qquad (2)$$

Here, $S_0$ is the initial substance concentration, t is the time, and A is a constant.

In this manner, there is a large amount of reaction at the pre-reaction portion and around the periphery of the reactor and because of this, the heat response increases the amount of heat generated by the heat response and so there is a large cooling load at these portions.

In addition, the gas generation system has a large gas hold up in the later stages as a result of the larger amount of gas generation in the pre-reaction portion, and this is a cause of lowered efficiency. Moreover, this also makes it easier for the carriers to block in the post reaction portion.

One method of alleviating these problems is to provide reactors a which is provided with a plural number of stages a, each of which is provided with a carrier b which is provided with a built-in gas removal chamber c, as indicated in FIG. 4. (see for example JP-U-61-7 7800) Moreover, the stages of the reactors a are mutually connected at their top portions and their bottom portions by a pipe d and pump e, and in addition, the stages of the reactors a are also mutually connected by a separate pump f along a pipe d so that gas removal from the reactors a can be performed and so that there is no gas hold-up in the later stages.

However, in such a structure, there is another pump f between each of the reactors a and so it is necessary to perform flow adjustment for the pumps e and f to control the level of the liquid between the tanks a and a and in addition, because there are a plural number of tanks a, there is also the problem of there being a large heat-conductive surface.

In the light of these problems, the present invention has as an object the provision of a bioreactor of the double-cylinder type in which the heat-transfer surface is enlarged at the pre-reaction portion of the reaction tank, and in which the gas removal is performed to the maximum extent possible, and which has a simple structure and good operability.

2

## DISCLOSURE OF INVENTION

The present invention is for an upright type of cylindrical reactor which has a double-layered structure divided into an internal cylinder inside an outer tank forming an outer communicating path and an inner communicating path. Then a liquid inlet at a lower portion of the outer communicating path is provided along with a liquid outlet at a lower portion of an inner side communicating path. Moreover, an outer periphery of said outer cylinder is provided with a cooling jacket and an upper surface of an inner-outer side communicating path in the vicinity of the top of an outer cylinder forms a gas removal chamber, with outer side communicating path and inner side communication path being filled with carriers to which the biological catalyst adheres.

## BRIEF DESCRIPTION OF DRAWINGS

FIG. 1 is a longitudinal sectional view of a first embodiment of a double-cylinder type of bioreactor according to the present invention.

FIG. 2 is a longitudinal sectional view of a variation of the first embodiment of a double-cylinder type of bioreactor according to the present invention.

FIG. 3 is a diagram describing the characteristics of the bioreactor.

FIG. 4 is a diagram describing a conventional bioreactor.

## BEST MODE FOR CARRYING OUT THE INVENTION

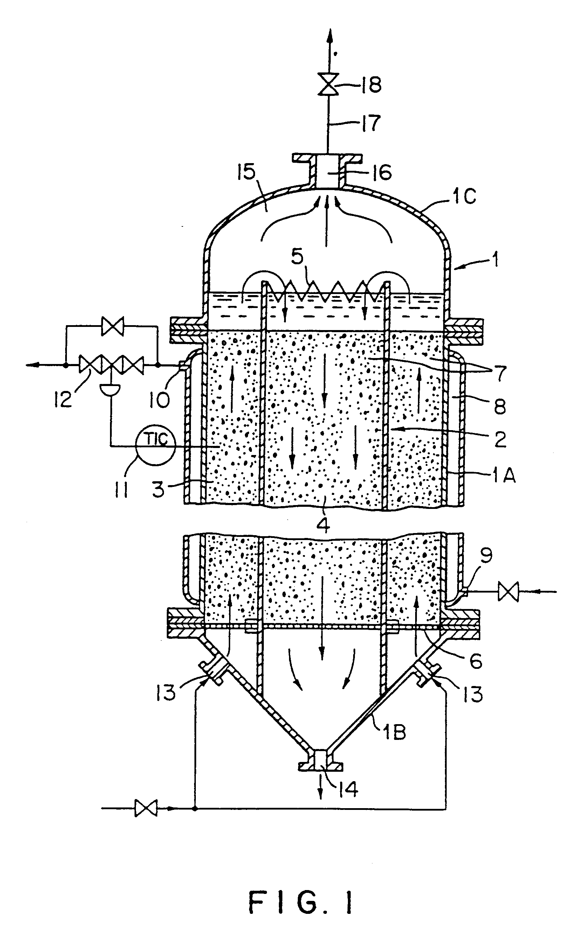

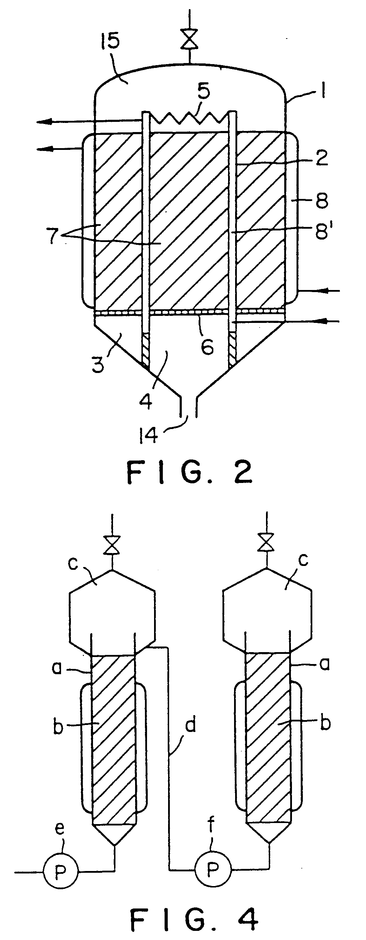

The following is a description of the embodiments indicated in FIG. 1 and FIG. 2 for the double-cylinder type of bioreactor according to the present invention.

FIG. 1 and FIG. 2 indicate a longitudinal sectional view of a first embodiment of a double-cylinder type of bioreactor according to the present invention, in which the reactor is a reactor for aerophobic enzymes. Therefore, the reactor has an outer tank 1 and an inner cylinder 2 standing inside the outer tank 1.

The outer tank 1 has an upright, sealed cylindrical shape and comprises a middle cylinder portion 1A, a bottom portion 1B having the shape of an inverted cone in section and sealed at its bottom portion, and a lid 1C sealing a top portion of the middle cylinder portion 1A. These for bolt connected at flanges (not indicated in the figure) to form a sealed structure.

The inner cylinder 2 stands on the bottom portion 1B and because of this inner cylinder 2, the inside of the tank is divided into an outer communicating path 3 formed with the inner surface of the outer tank 1, and an inner communicating path 4 formed with the inside of the inner cylinder 2. The upper end of the inner cylinder 2 extends to a height so that there is a gap of a predetermined size with respect to the lower surface of the lid 1C, and the edge at the upper end of the inner cylinder 2 is formed with a sawtooth shape to that it forms a weir 5.

The contact surface between the bottom portion 1B and the middle cylinder portion 1A of the outer tank 1 and the position where the surfaces of the inner cylinder 2 come into contact is provided with plate 6 of punched metal or mesh or the like and having many holes, and is filled with carriers 7 to which the biological catalyst is made to adhere.

The outer periphery of the middle cylinder portion 1A of the outer tank 1 is formed into a cooling jacket 8 and the lower portion is provided with a cooling medium inlet 9 while the upper portion is provided with a cooling medium outlet 10. This cooling medium outlet 10 is provided with a valve apparatus 12 to adjust the amount of flow of the cooling medium, and the temperature inside the outer communicating path 3 is detected by a temperature control means 11 and controlled.

In addition, the bottom portion 1B of the outer tank 1 is provided with a liquid inlet 13 to the outer communicating path 3, and a liquid outlet 14 from the inner communicating path 4.

The inside at the upper portion of the outer tank 1 is formed into a gas removal chamber 15 and this is connected to a gas removal tube 17. Along this gas removal tube 17 is provided a valve 18 in order to adjust the amount of gas removal and therefore adjust the pressure inside the tank 1.

The following is a description of the operation of a first embodiment of a double-cylinder type of bioreactor according to the present invention.

First, the liquid the flows in through the liquid inlet 13 enters the outer communicating path 3 and the reaction proceeds while the liquid passes through the carriers 7 filled inside the outer communicating path 3, and the heat generated by this reaction causes undergoes heat exchange due to the cooling jacket 8 on the outer periphery of the outer tank 1 and is sufficiently cooled.

The liquid that rises through the outer communicating path 3 overflows over the spin chuck 5 and into the inner communicating path 4 at the upper end of the inner cylinder 2 so that it is exposed to the air in the space inside the gas removal chamber 15 and separated into air and liquid, with only the liquid flowing into the inner communicating path 4. In addition, the reactions proceeds to the post-stage while the liquid flows down inside the inner communicating path 4 and the liquid flows from the liquid outlet 14 when the reaction has finished.

On the other hand, the air that is separated in the gas removal chamber 15 is discharged from the mounting table 16 and via the gas removal tube 17 and the valve 18.

FIG. 2 is an embodiment for processing in cases where the reaction temperature inside the inner communicating path 4 becomes hot because of an insufficient heat-conductive area afforded by only the cooling jacket 8 being provided on the outer periphery of the outer tank 1. In this embodiment, the inner cylinder 2 is formed into a two-layered structure having a cooling jacket 8' on the inside, to and from which cooling medium is supplied and discharged. The other parts of the configuration are as indicated in FIG. 1, are indicated with the same numerals, and the description of them is omitted.

In addition, in cases where it is difficult to adjust the level of the liquid in the inner communicating path 4, the periphery of the upper portion of the inner cylinder 2 can be provided with many holes of small diameter, and the outer communicating path 3 and the inner communicating path 4 linked so that it is easier to maintain the status where the liquid is filled up to the overflow line.

Furthermore, to make the reactor into an aerophilic reactor, an air feed pipe can be introduced into the bottom portion of the plate 6 with many holes and air fed in the direction of the carriers 7.

As has been described above, according to the present invention, it is possible to greatly increase the heat-conductive area in an outer side communicating path of a pre-stage reaction and therefore effectively perform the removal of reaction heat. Moreover, liquid is made to overflow from the outer communicating path to an inner communicating path and so by selecting the ratio of areas between the inner communicating path and the outer communicating path, it is possible to easily achieve a balance of flow between the inside and the outside. Because of this, it is not necessary to perform the difficult flow control that was conventionally necessary and the operation becomes extremely simple.

INDUSTRIAL APPLICABILITY

A double-cylinder bioreactor according to the present invention can have a heat-conductive area equal to the size of the pre-stage reaction portion of the reactor and for gas removal to be performed for the prestage reaction portion, and also have a simple structure and operation. For these reasons, the present invention can be applied to bioreactors that have a reactor that produces a predetermined biological substance through catalysis with a biological catalyst. The range of application of the present invention is diverse, and includes reactors for either aerophobic or aerophilic enzymes.

**Claims**

1.  An upright type double cylinder bioreactor, comprising:
    a double-wall structure having an inner cylinder (2) inside an outer tank (1) forming an outer communicating path (3) and an inner communicating path (4), the lower portion of said outer communicating path (3) being provided with a liquid inlet (13) and the lower portion of said inner communicating path (4) being provided with a liquid outlet (14), an outer periphery of said outer tank (1) being provided with a cooling jacket (8) and an upper surface of said inner and outer communicating paths (3, 4) in the vicinity of the top of said outer tank (1) forming a gas removal chamber (15) and said outer communicating path (3) and said inner communicating path (4) being filled with carriers (7) to which a biological catalyst adheres.

2.  An upright type double cylinder bioreactor of claim 1, wherein:
    an inner portion of said inner cylinder (2) is formed as a cooling jacket (8') to produce a double-wall structure.

3.  An upright type double cylinder bioreactor of claim 1, wherein:
    a weir (5) is formed with a sawtooth shape around the upper edge of said inner cylinder (2).

4.  An upright type double cylinder bioreactor of claim 1, wherein:
    a plural number of small-diameter holes are provided around the upper end portion of said inner

4

EP 0 381 776 B1

cylinder (2).

**Patentansprüche**

1. Doppelzylinder-Bioreaktor aufrechter Bauart, enthaltend:
   eine Doppelwandstruktur, die einen inneren Zylinder (2) innerhalb eines äußeren Tanks (1) unter Ausbildung eines äußeren kommunizierenden Pfads (3) und eines inneren Kommunizierenden Pfads (4) aufweist, wobei der untere Abschnitt des äußeren kommunizierenden Pfads (3) mit einem Flüssigkeitseinlaß (13) versehen ist und der untere Abschnitt des inneren kommunizierenden Pfads (4) mit einem Flüssigkeitsauslaß (14) versehen ist, am Außenumfang des äußeren Tanks (1) ein Kühlmantel (8) vorgesehen ist und eine obere Oberfläche des inneren und äußeren kommunizierenden Pfads (3,4) in der Nähe des oberen Endes des äußeren Tanks (1) eine Gasentfernungskammer (15) bildet und der äußere kommunizierende Pfad (3) und der innere kommunizierende Pfad (4) mit Trägern (7) gefüllt sind, an denen ein biologischer Katalysator haftet.

2. Doppelzylinder-Bioreaktor aufrechter Bauart nach Anspruch 1, bei dem:
   ein innerer Abschnitt des inneren Zylinders (2) als ein Kühlmantel (8') ausgebildet ist, um eine Doppelwandstruktur vorzusehen.

3. Doppelzylinder-Bioreaktor aufrechter Bauart nach Anspruch 1, bei dem:
   ein Wehr (5) sägezahnförmiger Gestalt rund um den oberen Rand des inneren Zylinders (2) ausgebildet ist.

4. Doppelzylinder-Bioreaktor aufrechter Bauart nach Anspruch 1, bei dem:
   eine Vielzahl Löcher kleinen Durchmessers rund um den oberen Endabschnitt des inneren Zylinders (2) vorgesehen ist.

**Revendications**

1. Bioréacteur vertical du type à double cylindre, comprenant :
   une structure à double paroi comprenant un cylindre intérieur (2) à l'intérieur d'une cuve extérieure (1) formant un trajet extérieur de communication (3) et un trajet intérieur de communication (4), la partie inférieure dudit trajet extérieur de communication (3) étant dotée d'une entrée pour des liquides (13) et la partie inférieure dudit trajet intérieur de communication (4) étant dotée d'une sortie (14) pour des liquides, une périphérie extérieure de ladite cuve extérieure (1) étant équipée d'une enveloppe de refroidissement (8) et une surface supérieure desdits trajets intérieur et extérieur de communication (3, 4) au voisinage du sommet de ladite cuve extérieure (1) constituant une chambre d'élimination des gaz (15) et ledit trajet extérieur de communication (3) ainsi que ledit trajet intérieur de communication (4) étant remplis de porteurs (7) auxquels un catalyseur biologique adhère.

2. Bioréacteur vertical du type à double cylindre selon la revendication 1, dans lequel :
   une partie intérieure dudit cylindre intérieur (2) est constituée comme une enveloppe de refroidissement (8') pour créer une structure à double paroi.

3. Bioréacteur vertical du type à double cylindre selon la revendication 1, dans lequel :
   un déversoir (5) est constitué avec une forme en dents de scie autour du bord supérieur dudit cylindre intérieur (2).

4. Bioréacteur vertical du type à double cylindre selon la revendication 1, dans lequel :
   un grand nombre d'orifices de petit diamètre sont formés autour de la partie de l'extrémité supérieure dudit cylindre intérieur (2).

5

F I G. 1

FIG. 2

FIG. 4

FIG. 3